(19) Europäisches Patentamt
European Patent Office
Office européen des brevets

(11) **EP 3 329 257 B1**

(12) **FASCICULE DE BREVET EUROPEEN**

(45) Date de publication et mention de la délivrance du brevet:
**12.07.2023 Bulletin 2023/28**

(21) Numéro de dépôt: **16763889.9**

(22) Date de dépôt: **28.07.2016**

(51) Classification Internationale des Brevets (IPC):
**G01N 21/64** *(2006.01)*    **G01N 21/27** *(2006.01)*
**G01N 33/14** *(2006.01)*

(52) Classification Coopérative des Brevets (CPC):
**G01N 21/64; G01N 21/278; G01N 33/146;**
G01N 21/6486; G01N 2021/6421; G01N 2021/6497;
G01N 2201/0221

(86) Numéro de dépôt international:
**PCT/FR2016/051971**

(87) Numéro de publication internationale:
**WO 2017/021629 (09.02.2017 Gazette 2017/06)**

(54) **ENSEMBLE COMPRENANT UN CONTENANT ET UN DISPOSITIF PORTABLE POUR LE CONTRÔLE D'UNE BOISSON ALCOOLISÉE À TRAVERS LEDIT CONTENANT, SYSTÈME ET PROCÉDÉ ASSOCIÉS**

ENSEMBLE UMFASSEND EINEN BEHÄLTER SOWIE EINE TRAGBARE VORRICHTUNG ZUM TESTEN EINES ALKOHOLISCHEN GETRÄNKS DURCH DEN BEHÄLTER, ZUGEHÖRIGES SYSTEM UND VERFAHREN

ENSEMBLE COMPRISING A CONTAINER AND A PORTABLE DEVICE FOR TESTING AN ALCOHOLIC BEVERAGE THROUGH THE CONTAINER, ASSOCIATED SYSTEM AND METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.07.2015 FR 1557433**

(43) Date de publication de la demande:
**06.06.2018 Bulletin 2018/23**

(60) Demande divisionnaire:
**22179248.4 / 4 095 517**

(73) Titulaires:
- **Martell & Co**
  **16100 Cognac (FR)**
- **Centre National de la Recherche Scientifique**
  **75016 Paris (FR)**
- **Université de Bordeaux**
  **33000 Bordeaux (FR)**
- **Institut Polytechnique de Bordeaux**
  **33400 Talence (FR)**

(72) Inventeurs:
- **BRUNERIE, Pascal**
  **94440 Santeny (FR)**
- **GOURET, Katia**
  **16200 Les Metairies (FR)**
- **FIL, Benoit**
  **16100 Louzac (FR)**
- **VERGER, Stephane**
  **16100 Cognac (FR)**
- **BRUNEEL, Jean-Luc**
  **33650 Saint Selve (FR)**
- **GUILLAUME, François**
  **33140 Villenave d'Ornon (FR)**
- **BRUNEEL DELHAYE, Caroline**
  **33650 Saint Selve (FR)**

(74) Mandataire: **Germain Maureau**
**12, rue Boileau**
**69006 Lyon (FR)**

(56) Documents cités:
| | |
|---|---|
| WO-A2-02/33404 | WO-A2-2005/001451 |
| WO-A2-2005/001451 | WO-A2-2014/184506 |
| WO-A2-2014/184506 | US-A1- 2002 105 642 |
| US-A1- 2002 105 642 | US-A1- 2007 023 521 |

US-A1- 2015 009 490

**Description**

La présente invention se rapporte au domaine de l'authentification d'un contenu telle qu'une boisson, notamment une boisson alcoolisée. Plus précisément, l'invention concerne un ensemble comprenant un contenant et un dispositif et un procédé permettant d'authentifier une boisson à travers les parois du contenant, plus particulièrement lorsque ce contenant est une bouteille.

Les coûts de production et de commercialisation des boissons alcoolisées de haut de gamme, telles que les vins de grand cru et les spiritueux, aboutissent à des prix de vente élevés. Ces prix élevés génèrent, comme souvent pour les produits de luxe, de la fraude ainsi que des actes de contrefaçon. Ainsi, on observe régulièrement l'apparition sur le marché de bouteilles contrefaites, c'est-à-dire présentant l'apparence extérieure d'une bouteille authentique, mais remplie d'une boisson de qualité très inférieure à la qualité du produit original. Ce phénomène de contrefaçon est particulièrement important pour les vins et spiritueux de haut de gamme, comme par exemple le cognac.

Pour des raisons économiques, de sécurité et d'image de marque, les producteurs concernés luttent énergiquement contre ces actes de fraude et de contrefaçon. Cette lutte se traduit notamment par la fabrication de bouteilles comportant des dispositifs anti-contrefaçon de plus en plus évolués, ayant pour but d'empêcher la reproduction exacte d'une bouteille et/ou sa réutilisation.

Toutefois, les contrefacteurs déploient également des efforts constants, et sont capables de reproduire des bouteilles authentiques avec une fidélité telle qu'il devient très difficile de distinguer une bouteille contrefaite d'une bouteille authentique. Dans d'autres cas, les contrefacteurs récupèrent des bouteilles authentiques usagées, qui sont alors remplies avec un produit non authentique.

Les documents WO2005/001451A2 et US2015/0009490A1 décrivent des systèmes portables pour identifier et/ou authentifier une substance chimique, notamment une boisson alcoolisée, à travers un contenant par spectroscopie de fluorescence.

Le document US2007/0023521A1 décrit un dispositif pour encoder l'emballage d'un produit. Le document WO2014/184506A2 décrit un dispositif d'authentification d'un contenu dans un contenant. Le document WO02/33404A2 décrit un dispositif pour évaluer l'état du vin dans une bouteille. Le document US2002/0105642A1 décrit un procédé pour évaluer la qualité d'un matériau optique.

Il existe par conséquent un besoin pour permettre de lever tout doute sur l'authenticité d'une bouteille, en authentifiant directement le contenu lui-même. Bien entendu, comme il n'est pas possible d'ouvrir chaque bouteille dont l'authenticité serait douteuse, une telle authentification doit pouvoir se faire à travers les parois de la bouteille.

En outre, cette question d'authentification se pose à des endroits variés, liés aux différents acteurs de la chaîne de distribution (revendeurs, distributeurs, etc.). La diversité de ces acteurs, et leur éloignement potentiel des lieux de production font qu'il existe un besoin pour un dispositif d'authentification qui soit portable, simple d'utilisation et peu coûteux, ainsi que pour un procédé d'authentification qui soit fiable et performant, notamment en termes de rapidité.

La présente invention a pour objectif de répondre à ces besoins.

Elle concerne à cet effet un ensemble comprenant un contenant et un dispositif, ledit ensemble étant tel que défini dans la revendication 1.

En utilisant le phénomène de fluorescence, le dispositif conforme à l'ensemble de l'invention permet de réduire la puissance de la source lumineuse d'excitation par rapport aux dispositifs connus, en particulier ceux utilisant l'effet Raman (l'intensité de la lumière émise dans le processus de fluorescence est en effet environ un million de fois plus intense que la diffusion Raman). Le dispositif conforme à l'ensemble de l'invention comporte une source lumineuse unique, ce qui participe à la simplicité et à la portabilité du système. En outre, en se limitant à des longueurs d'onde supérieures à 350 nanomètres, on limite l'influence des molécules d'eau et d'éthanol (très largement majoritaires en concentration dans une boisson alcoolisée telle qu'un vin ou un spiritueux). En effet, les molécules d'eau et d'éthanol n'absorbent pas le rayonnement lumineux pour des longueurs d'onde supérieures à 200 nanomètres. Par ailleurs, en prévoyant un montage dit « en réflexion », on limite l'atténuation du rayonnement de fluorescence. Au surplus, l'angle d'incidence de 180° entre le rayon émis et le rayon de fluorescence réfléchi permet de minimiser les effets indésirables dus à la paroi du contenant, puisque l'épaisseur traversée sera minimale. Enfin, en prévoyant un dispositif de positionnement, on s'assure que la focalisation du faisceau émis se fera à l'endroit désiré, sans qu'aucun réglage ne soit nécessaire. La répétabilité et la reproductibilité des mesures sont ainsi optimisées.

Dans une réalisation, le diviseur de faisceau est un filtre dichroïque, notamment un filtre dichroïque passe-haut (relativement aux longueurs d'onde).

Dans une réalisation, le dispositif de filtrage comporte un filtre coupe-bande de type Notch et/ou un filtre passe-haut (relativement aux longueurs d'onde).

Dans une réalisation, le module de spectrométrie est relié au dispositif de filtrage par l'intermédiaire d'une fibre optique.

Le dispositif de positionnement comporte une surface de contact configurée pour être complémentaire de la surface externe du contenant.

Dans une réalisation, le dispositif comporte un dispositif d'affichage, permettant notamment d'afficher le résultat de la comparaison entre le spectre mesuré et le spectre de référence.

Le dispositif comporte un boitier duquel est solidaire le dispositif de positionnement.

Dans une réalisation, le dispositif de positionnement est connecté au boitier par l'intermédiaire d'une liaison flexible, la lentille de focalisation étant intégrée au dispositif de positionnement, la liaison flexible comportant une fibre optique pour transmettre les faisceaux lumineux traversant la lentille de focalisation.

Dans une réalisation, le module d'analyse et le dispositif d'affichage sont inclus dans un dispositif portable annexe, tel qu'une tablette informatique ou un téléphone mobile.

Dans une réalisation, le dispositif comporte des moyens de connexion à une base de données distante.

L'invention concerne également un système de contrôle d'une boisson alcoolisée, le système comportant un ensemble tel que défini ci-dessus et une base de données stockée dans un serveur distant du dispositif.

Dans une réalisation, la base de données comporte un ou plusieurs spectres de référence pouvant être téléchargés par le dispositif.

L'invention concerne en outre un procédé de contrôle d'une boisson alcoolisée à travers un contenant au moins partiellement transparent, ledit procédé étant défini dans la revendication 11.

Le procédé de contrôle conforme à l'invention présente de nombreux avantages. Grâce à l'étape de normalisation du spectre mesuré, par exemple par rapport au maximum d'intensité du spectre de référence, on s'affranchit des dispersions dues à la température du contenu contrôlé et aux variations de caractéristiques du contenant (notamment les caractéristiques dimensionnelles). Cette étape de normalisation permet également de collecter avec une source d'excitation de faible puissance un signal de qualité suffisante pour être correctement analysé. En outre, l'étape de comparaison du spectre mesuré avec le spectre de référence, qui implique le calcul d'un facteur de ressemblance, nécessite des capacités de calcul très limitées, capacités aujourd'hui disponibles sur un téléphone mobile de type « smartphone ».

Dans une réalisation, le spectre de fluorescence est acquis en utilisant une source émettant un faisceau monochromatique de longueur d'onde comprise entre 350 et 650 nanomètres.

Dans une réalisation, le facteur de ressemblance est calculé sur une plage de longueurs d'onde prédéterminée, par exemple une plage de longueurs d'onde comprises entre 550 et 650 nanomètres.

Dans une réalisation, le facteur de ressemblance est calculé par la méthode dite des moindres carrés ou par un algorithme du type de l'algorithme de Hausdorff.

Dans une réalisation, la boisson est déterminée comme non authentique si le facteur de ressemblance est supérieur à 20.

Dans une réalisation, le spectre de référence est obtenu à partir de la mesure du spectre de fluorescence mesuré sur un échantillon d'une pluralité de bouteilles authentiques.

Dans une réalisation, le procédé comporte une étape de téléchargement du spectre de référence depuis une base de données distante.

Le procédé est mis en oeuvre à l'aide d'un ensemble ou un système tel que défini plus haut.

L'invention sera mieux comprise à la lecture de la description ci-après, faite en référence aux dessins annexés, parmi lesquels :

- les figures 1 et 2 représentent un ensemble conforme à l'invention en cours d'utilisation, respectivement vu de côté et vu de dessus ;
- la figure 3 représente le dispositif de la figure 1, vu de face ;
- la figure 4a est un schéma de principe d'un ensemble conforme à l'invention ;
- la figure 4b est un détail de la figure 4a ;
- la figure 5 montre une variante de réalisation du dispositif de la figure 4a ;
- les figures 6 et 7 sont des courbes correspondant respectivement à un spectre de référence et à un spectre mesuré ;
- la figure 8 est un diagramme détaillant les étapes du procédé conforme à l'invention.

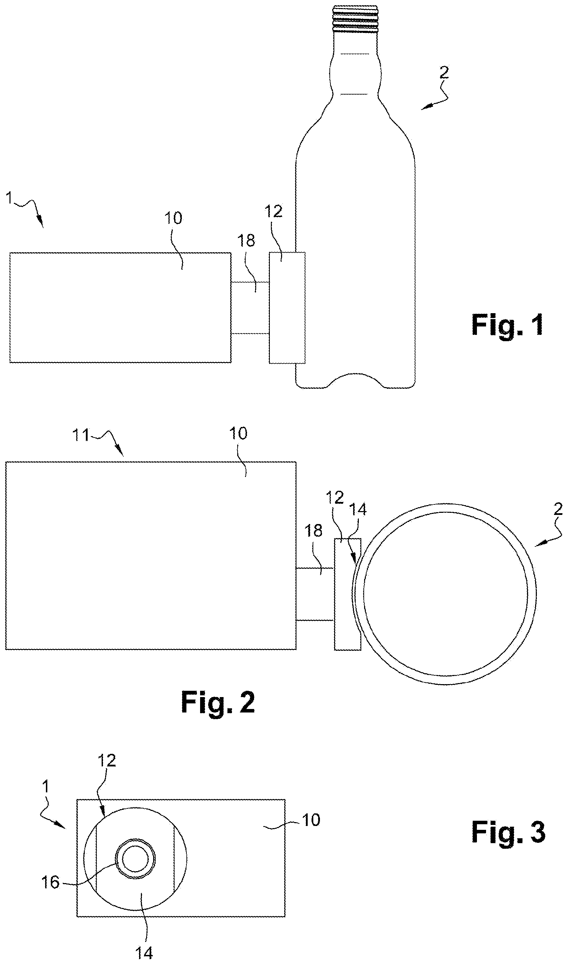

La figure 1 montre un dispositif 1 portable d'un ensemble conforme à l'invention, ainsi qu'un contenant dont le contenu doit être authentifié, dans l'exemple une bouteille 2 contenant une boisson alcoolisée. Le dispositif comporte un boitier 10, dans l'exemple de forme parallélépipédique. Le dispositif 1 comporte un dispositif de positionnement de la bouteille 2, ou cale 12 de positionnement. La cale 12 forme une saillie depuis l'une des faces du boitier.

Comme visible sur la figure 2, qui est une vue de dessus du dispositif 1 et de la bouteille 2, la cale 12 présente une surface de contact 14 de forme complémentaire du contenant. Dans l'exemple, la surface de contact 14 présente ainsi une forme concave adaptée à la forme convexe de la bouteille 2.

Comme montré sur la figure 3, la cale 12 présente une ouverture 16 formant un passage permettant que les rayons lumineux émis par le dispositif 1 puissent traverser la cale 12. La cale 12 est solidaire du boitier 10 par l'intermédiaire d'un support 18, ce support 18 présentant également un passage traversant pour les rayons lumineux émis ou reçus par le dispositif 1.

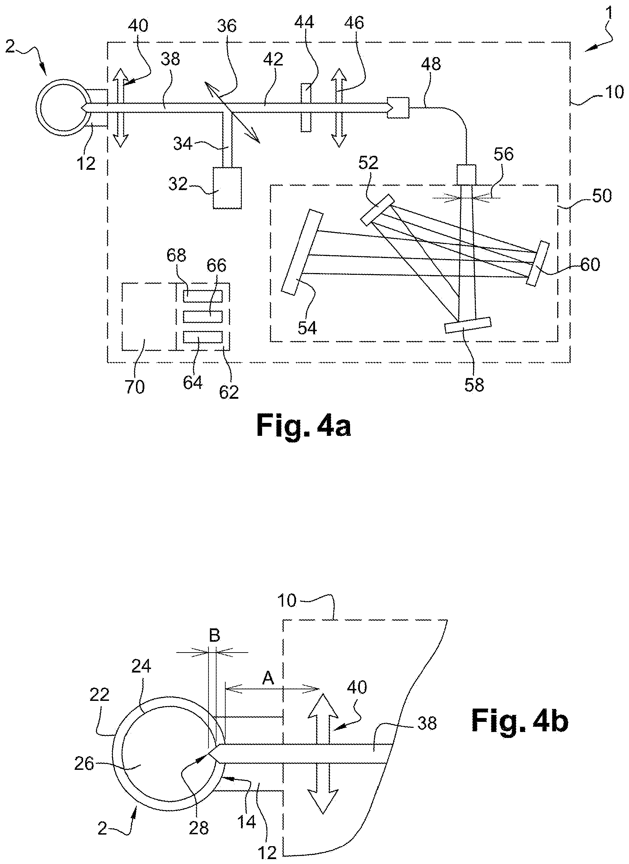

La figure 4a est un schéma de principe des composants d'un exemple de dispositif de l'ensemble conforme à l'invention. Sur cette figure, pour des raisons de clarté, une bouteille 2 est représentée, et le boitier 10 du dispositif 1 est représenté en pointillé. Le dispositif 1 représenté sur la figure 4a comporte une source lumineuse 32 d'excitation unique, émettant un faisceau monochromatique de longueur d'onde $\lambda_0$. Dans l'exemple la source lumineuse 32 est une diode laser.

La longueur d'onde $\lambda_0$ de la source lumineuse 32 est comprise entre 350 nanomètres et 650 nanomètres. Cette plage permet d'obtenir que le faisceau lumineux émis génère une forte émission de fluorescence des molécules permettant de caractériser la boisson alcoolisée (c'est-à-dire les molécules autres que celles de l'eau et de l'éthanol). De plus, dans cette plage, le faisceau émis par la source lumineuse 32 n'est que faiblement atténué par le verre de la bouteille 2. Toujours dans cette plage, l'émission de fluorescence du contenu de la bouteille 2 n'est que très faiblement atténué par le verre de la bouteille 2.

Le faisceau lumineux 34 issu de la source lumineuse 32 est dirigé vers un diviseur de faisceau 36. Le diviseur de faisceau 36, dans l'exemple un filtre dichroïque, est orienté selon un angle de 45° par rapport à la direction du faisceau lumineux 34 émis par la source 32. Ainsi, le faisceau émis par la source 32 est réfléchi par le diviseur de faisceau 36 et le faisceau réfléchi 38 est dirigé vers une lentille de focalisation 40, dans l'exemple une lentille doublet achromatique. La lentille de focalisation 40 est située à une distance A de la bouteille 2.

Comme visible sur la figure 4b, qui représente un détail de la figure 4a, la bouteille 2, dans l'exemple de section transversale circulaire, comporte une paroi délimitée par une surface externe 22 et une surface interne 24. La bouteille 2 contient une boisson 26 devant être authentifiée. La cale 12 est en contact avec la surface externe 22 de la bouteille 2. Comme mentionné plus haut, la cale permet de positionner la bouteille 2 de telle manière que la lentille de focalisation 40 soit à une distance A déterminée de la surface externe 22 de la bouteille 2. En outre, la cale 12, par la forme de sa surface de contact 14, adaptée pour coopérer avec la forme de la surface externe 22 de la bouteille, permet de positionner la bouteille 2 de telle sorte que le faisceau lumineux traversant la lentille de focalisation sera dirigé vers la surface externe 22 de la bouteille 2 selon une direction normale à cette surface. Dans l'exemple, la surface de contact 14 de la cale 12 est de forme concave et est adaptée pour coopérer avec la forme extérieure convexe de la bouteille 2. On notera à cet égard que la cale 12, et en particulier la forme de la surface de contact 14, sera adaptée dans le cas où le contenant n'est pas de forme circulaire. Quelle que soit la forme du contenant considéré, on s'assurera que la forme de la surface de contact 14 permet de positionner la cale 12 sur le contenant de telle sorte que le faisceau lumineux traversant la lentille de focalisation 40 soit dirigé vers la surface externe du contenant selon une direction normale à cette surface. Afin d'adapter facilement la cale 12 de positionnement à chaque type de contenant, on pourra bien entendu prévoir que la cale 12 soit fixée de manière amovible au boitier 10. On pourra par ailleurs envisager que la cale 12 soit connectée au boitier par l'intermédiaire d'une liaison flexible pour faciliter le positionnement de la cale 12 sur un contenant. Dans ce cas, la lentille de focalisation sera déportée du boitier et solidaire de la cale 12 de positionnement. A cet effet, la liaison flexible entre le boitier et l'ensemble formé par la cale 12 de positionnement et par la lentille de focalisation comportera une fibre optique pour transmettre les faisceaux lumineux depuis le boitier vers la lentille de focalisation et inversement.

L'espacement assuré par la cale 12 entre la lentille de focalisation 40 et la bouteille 2 sera déterminé de telle sorte que le faisceau réfléchi 38 sera focalisé à l'intérieur de la bouteille 2, au sein de la boisson 26, mais au plus près de la surface interne 24. La figure 4b montre ainsi le point de focalisation 28 situé au sein de la boisson 26, à une distance B de la paroi de la bouteille 2 (plus précisément : à une distance B de la surface interne 24 de la paroi de la bouteille 2). Plus la distance B est faible, plus on minimise le trajet du faisceau émis et celui du rayonnement de fluorescence, et plus on limite l'atténuation de ces deux signaux. La distance B sera avantageusement inférieure à 1 millimètre, et sa valeur sera de préférence de l'ordre de quelques centaines de micromètres ou moins.

Le signal de fluorescence 42 induit dans le contenu de la bouteille 2 par le faisceau lumineux est collecté par la lentille de focalisation 40, par rétro-émission, et est dirigé vers le diviseur de faisceau 36. Le diviseur de faisceau 36 laisse passer le signal de fluorescence qui est dirigé vers un module de spectrométrie 50.

Avantageusement, le dispositif 1 comporte un ou plusieurs dispositifs de filtrage, permettant de filtrer le signal de fluorescence 42 avant que ce dernier soit transmis au module de spectrométrie. Le but de ce filtrage est notamment d'éliminer la composante du signal collecté par la lentille de focalisation 40 lié au faisceau lumineux émis par la source 32. Dans ce but on prévoit dans l'exemple un filtre coupe-bande 44 ou filtre « notch ». L'intervalle de fréquences non transmises par le filtre coupe-bande sera proche de la longueur d'onde du faisceau lumineux monochromatique émis par la source 32. La bande non transmise aura par exemple une largeur d'environ 10 nanomètres, et sera centrée sur une longueur d'onde supérieure de quelques nanomètres ou moins (par exemple 1 nanomètre) à la longueur d'onde $\lambda_0$ de la source laser.

On peut en outre prévoir qu'un filtrage additionnel, de type passe-haut (relativement aux longueurs d'onde), soit effectué par le diviseur de faisceau 36. Dans ce cas, le diviseur de faisceau 36 est un filtre dichroïque de type passe haut à forte pente de coupure (notamment un filtre de type « edge ») qui réfléchit le faisceau 34 vers la lentille de focalisation, et transmet le rayonnement émis par la boisson et son contenant vers le module de spectrométrie en éliminant les longueurs d'ondes inférieures à une valeur seuil correspondant à la longueur d'onde du faisceau monochromatique émis par la source 32 augmentée de 10 nm environ.

Dans l'exemple de la figure 4a, le signal de fluorescence 42, une fois filtré par le diviseur de faisceau 36 et par le filtre coupe-bande 44, est dirigé vers une lentille 46, dans l'exemple une lentille asphérique de focale très courte. Le faisceau transmis par la lentille 46 est dirigé vers une extrémité d'un segment de fibre optique 48, l'autre extrémité de ce segment étant reliée au module de spectrométrie 50. La fibre optique 48 de par sa flexibilité, permet d'optimiser le placement des composants au sein du boitier 10 et ainsi d'en limiter l'encombrement. En outre, celle-ci joue le rôle d'un trou confocal et limite ainsi la quantité d'informations transmise et donc analysée. Ainsi, des effets indésirables, en particulier ceux liés à la forme et à l'épaisseur de la bouteille ou ceux liés à l'éclairage extérieur, peuvent être minimisés. Par ailleurs, la fibre optique permet de s'affranchir également de l'effet d'astigmatisme, qui implique des variations de focalisation à l'entrée du module de spectrométrie en fonction des différentes longueurs d'ondes du rayonnement de fluorescence.

Dans une variante, la lentille 46 peut être remplacée par un objectif de grossissement.

Le module de spectrométrie 50 comporte un réseau de diffraction 52 permettant de diriger le signal diffracté vers un capteur 54, dans l'exemple un capteur de type CCD qui présente comme avantage de ne pas nécessiter de refroidissement. Dans l'exemple, le réseau de diffraction 52 est un réseau de diffraction réfléchissant. Le signal entrant dans le module de spectrométrie 50, via une fente d'entrée 56, est dirigé vers un premier miroir 58, qui est un miroir convexe. Le signal réfléchi par le réseau de diffraction 52 est dirigé vers un deuxième miroir 60, de type plan. Le deuxième miroir réfléchit le signal diffracté vers le capteur 54. Dans une variante, l'ensemble 52-58-60 peut être remplacé par un seul réseau de diffraction concave permettant d'améliorer la compacité du dispositif. Dans une autre variante, l'ensemble 52-58-60 peut être remplacé par un seul réseau de diffraction opérant en transmission.

Le capteur 54 fournit un signal correspondant à un spectre de fluorescence. Ce signal est transmis du module de spectrométrie 50 à un module d'analyse 62. Le module d'analyse 62 comporte notamment une unité de stockage 64, permettant de stocker en mémoire un signal en provenance du module de spectrométrie. L'unité de stockage 64 présente une capacité permettant de stocker un signal obtenu sur une certaine durée d'acquisition, par exemple de l'ordre de 150 millisecondes. Le module d'analyse 62 comporte en outre une unité de calcul 66, permettant de déterminer si le signal enregistré correspond à celui d'une boisson authentique. A cet effet, l'unité de calcul 66 effectue notamment une comparaison entre le signal enregistré et un signal de référence, de préférence selon un le procédé conforme à l'invention, qui sera décrit plus en détail ci-après. Le signal de référence pourra être préalablement stocké dans l'unité de stockage 64 et/ou téléchargé ou mis à jour depuis une base de données distante. A cet effet, le module d'analyse 62 comporte une unité de communication 68, notamment de type sans fil.

Le dispositif comporte enfin un dispositif d'affichage 70, permettant d'afficher le résultat du test. Le dispositif d'affichage 70 pourra consister en un écran d'affichage ou tout autre moyen, telle qu'une pluralité de voyants lumineux (réalisés par exemple avec des diodes électroluminescentes). On pourra par exemple prévoir que le résultat du test est donné selon l'une de deux voire trois possibilités : « BON », « MAUVAIS » et éventuellement « DOUTEUX ». Dans le cas d'une pluralité de voyants lumineux, on pourra ainsi prévoir trois voyants différents (notamment de couleurs différentes), chaque voyant correspondant s'allumant respectivement en fonction de l'une des trois possibilités mentionnées ci-dessus.

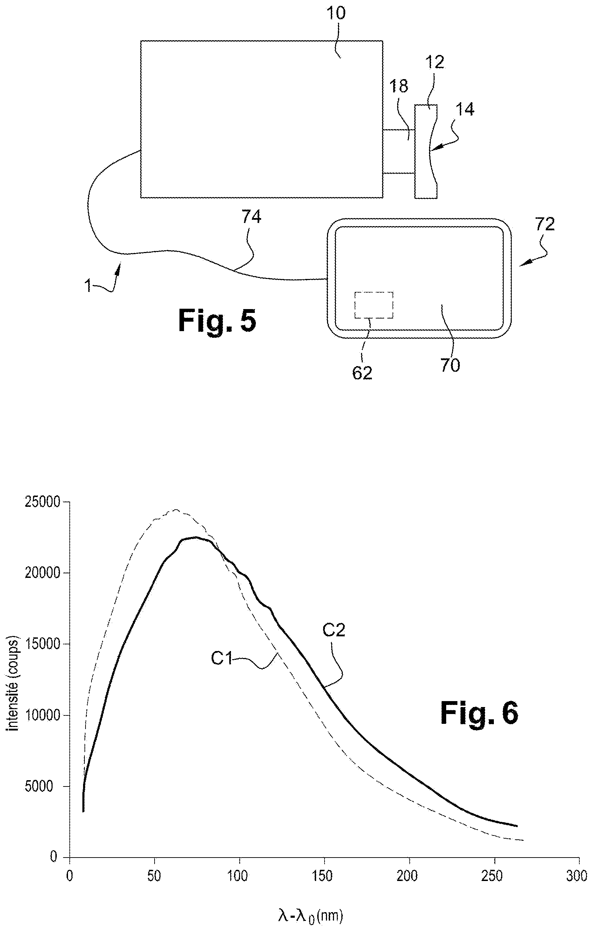

En variante, une partie ou la totalité du module d'analyse 62 et/ou du dispositif d'affichage 70 pourra être disposée dans un boitier distinct du reste du dispositif. La figure 5 représente une des configurations possibles, dans laquelle le module d'analyse 62 et le dispositif d'affichage 70 sont regroupés dans un dispositif externe, par exemple sous la forme d'un dispositif portable 72 tel qu'une tablette informatique ou un téléphone mobile. Dans un tel cas, on prévoit une liaison numérique 74, de type filaire ou sans-fil, entre le boitier 10 et le dispositif portable 72. Cette liaison permet de connecter le module de spectrométrie 50, disposé dans le boitier 10, au module d'analyse 62, situé dans le dispositif portable 72.

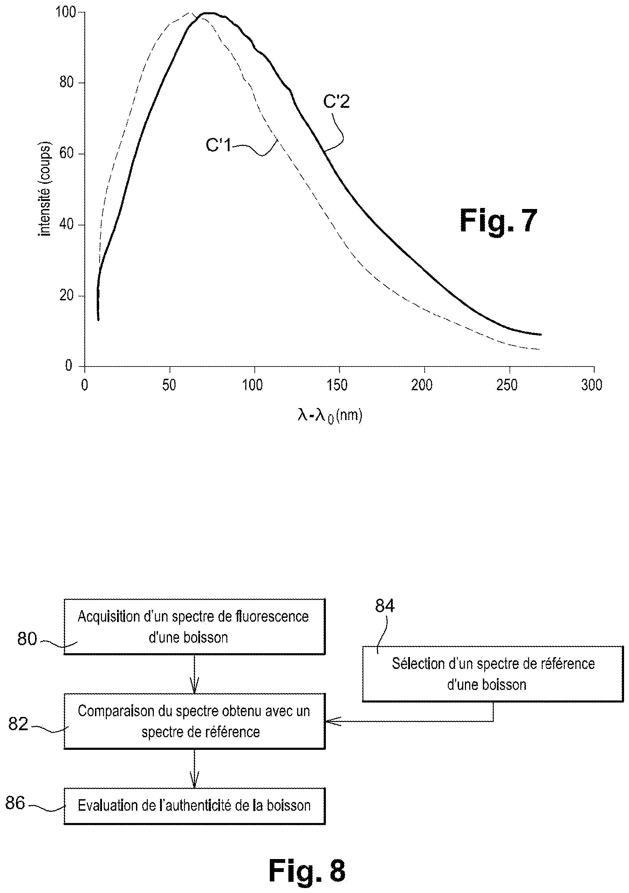

Les figures 6 et 7 montrent des exemples de spectre obtenus par le dispositif de l'ensemble conforme à l'invention, respectivement avant et après mise en oeuvre d'une étape de normalisation conforme au procédé selon l'invention.

La figure 6 montre une courbe C2 correspondant à un spectre de fluorescence obtenu pour un cognac authentique (contenu dans sa bouteille authentique d'origine) et une courbe C1 correspondant à un spectre obtenu pour un cognac non authentique (dans l'exemple un cognac de contrefaçon). Les deux courbes C1, C2 correspondent au signal de sortie du module de spectrométrie 50, et ont été obtenues avec une diode laser émettant un faisceau de longueur d'onde $\lambda_0$ proche de 530 nanomètres. On note que les deux courbes présentent un maximum, correspondant à une longueur d'onde de $\lambda_0$ +73 nanomètres pour le cognac authentique, et à $\lambda_0$ +58 nanomètres pour le cognac non authentique. En outre, l'intensité du maximum du spectre du cognac non authentique est supérieure à l'intensité du maximum du spectre du cognac authentique.

La figure 7 montre les deux spectres de la figure 6, après normalisation par rapport à l'intensité maximum du spectre du cognac authentique (en prenant pour base 100 l'intensité maximale du spectre du cognac authentique). Cette normalisation laisse subsister des différences importantes entre la courbe C'1 correspondant au profil normalisé du spectre du cognac non authentique et la courbe C'2 correspondant au profil normalisé du spectre du cognac authentique. Comme on le verra plus bas, ces différences sont exploitées par l'ensemble et le procédé conformes à l'invention afin de permettre la détection de boissons non authentiques à travers leur contenant.

On décrit ci-après le procédé de contrôle conforme à l'invention permettant l'authentification d'une boisson alcoolisée, dans le cadre d'un exemple appliqué à un cognac. Les principales étapes du procédé conforme à l'invention sont représentées à la figure 8. Ces étapes sont réalisées à l'aide d'un dispositif de l'ensemble conforme à l'invention, par exemple le dispositif 1 décrit ci-dessus.

Le procédé conforme à l'invention comporte une première étape consistant à acquérir un spectre mesuré de la boisson contrôlée. Cette étape d'acquisition 80 est réalisée notamment par le module de spectrométrie 50 du dispositif 1.

Le procédé comporte ensuite une étape de comparaison 82 du spectre mesuré avec un spectre de référence. Ce spectre de référence peut être stocké dans une mémoire du dispositif 1, par exemple une mémoire de l'unité de stockage 64, ou peut être téléchargé depuis une base de données à distance. De manière optionnelle, le procédé peut comporter une étape de sélection 84 du spectre de référence. Cette sélection peut s'opérer parmi plusieurs spectres de référence stockés en mémoire, ou parmi plusieurs spectres de référence disponibles dans la base de données distante. Cette sélection peut être effectuée automatiquement ou à la demande de l'utilisateur.

L'étape de comparaison 82 du spectre mesuré au spectre de référence comporte une sous-étape consistant en une normalisation du spectre mesuré, par rapport au maximum du spectre de référence (ou, alternativement, par rapport à l'intensité totale du spectre ou par rapport à l'aire sous le spectre). Cette normalisation présente plusieurs avantages. Cela permet notamment de s'affranchir des effets des variations de température. On sait que la température a une influence importante sur le phénomène de fluorescence. Mais les inventeurs ont constaté qu'un changement de température avait une influence importante sur l'intensité maximale du spectre de fluorescence, et une influence négligeable sur la forme du profil du spectre de fluorescence. En d'autres termes, après normalisation, des spectres d'une même boisson obtenus à des températures différentes sont superposables. Ainsi, le procédé conforme à l'invention permet de s'affranchir des variations de température lors de la collection du spectre de fluorescence. En particulier, cela permet de tenir compte de la différence entre la température à laquelle est collecté le spectre mesuré et la température à laquelle a été réalisé le spectre de référence. En outre, la normalisation du spectre mesuré permet de s'affranchir des effets des dispersions observées entre chaque bouteille d'un même type. On sait que même produits sur la même chaîne de fabrication, deux contenants « identiques » (comme par exemple des bouteilles en verre) présenteront toujours des dispersions, notamment des dispersions dimensionnelles (et donc d'épaisseur). Or, plus l'épaisseur de verre est importante, plus le faisceau émis par la source d'excitation sera atténué et, également, plus le rayonnement de fluorescence émis sera atténué dans la paroi du contenant. Or, les inventeurs ont constaté que cette atténuation avait une influence limitée sur le profil du spectre de fluorescence collecté, l'atténuation ayant surtout pour effet de diminuer l'intensité du spectre. Cette influence sur le profil du spectre est d'autant plus négligeable si la longueur du spectre collecté est inférieure à 700 nanomètres. Il est pour cela avantageux de limiter la plage de longueur d'onde utilisée pour mettre en oeuvre l'étape de comparaison, par exemple entre 550 et 650 nanomètres. Ainsi, le procédé conforme à l'invention permet de s'affranchir des variations d'épaisseur pour un même modèle de contenant.

L'étape de comparaison 82 du spectre mesuré au spectre de référence comporte une sous-étape de détermination d'un facteur R de ressemblance entre les courbes correspondant respectivement à un spectre mesuré pour une boisson donnée et au spectre de référence correspondant à cette boisson, dans l'exemple les courbes les courbes C'2 et C'1. Cette sous-étape permet ainsi de quantifier la ressemblance entre le spectre mesuré et le spectre de référence. Ce facteur R peut être calculé par exemple en utilisant la méthode des moindres carrés, selon la formule :

$$R_{1-2} = \sum_i \sqrt{\left(y_1(\lambda_i) - y_2(\lambda_i)\right)^2}$$

dans laquelle :

- $y_1(\lambda_i)$ est l'intensité du spectre mesuré (dans l'exemple la courbe C'1 de la figure 7) pour une longueur d'onde égale à $\lambda_i$ ;
- $y_2(\lambda_i)$ est l'intensité du spectre de référence (dans l'exemple la courbe C'2 de la figure 7) pour une longueur d'onde égale à $\lambda_i$.

On obtient ainsi une valeur nulle si les deux spectres sont totalement identiques. Plus le facteur R est élevé, plus les spectres comparés diffèrent.

Bien entendu, le facteur de ressemblance peut être calculé en utilisant tout autre algorithme adapté tel que par exemple l'algorithme de Hausdorff, selon la formule suivante :

$$R_{1-2} = \max \left\{ \sup_{x_2 \in y_2} \inf_{x_1 \in y_1} \delta(x_1, x_2), \sup_{x_1 \in y_1} \inf_{x_2 \in y_2} \delta(x_1, x_2) \right\}$$

Selon cette formule, on calcule pour chaque point $y_1(\lambda_i)$ du spectre $y_1(\lambda)$ sa plus faible distance aux points du spectre $y_2(\lambda)$. On choisit ensuite la plus grande distance calculée notée $\delta_{max}(y_1,y_2)$. On effectue alors le même calcul pour chaque point du spectre $y_2(\lambda)$ comparé au spectre $y_1(\lambda)$ en choisissant la plus grande distance calculée $\delta_{max}(y_2, y_1)$. Le facteur de ressemblance ou distance de Hausdorff sera alors la plus grande valeur de ces deux distances maximales retenues.

Quelle que soit la méthode utilisée, on obtient une valeur de R nulle si les deux spectres sont totalement identiques. De même, quelle que soit la méthode employée, plus le facteur R est élevé, plus les spectres comparés diffèrent.

La valeur de R sera calculée sur au moins une centaine de points, avantageusement plusieurs centaines. Dans l'exemple, le nombre de points pris en compte est de l'ordre de 600.

La valeur de R est utilisée lors de l'étape d'évaluation 86 de l'authenticité de la boisson. Cette étape permet de déterminer, sur la base de la valeur calculée du facteur R de ressemblance si la bouteille contrôlée contient une boisson authentique ou non. Par exemple, le contenu testé sera déclaré « mauvais » si le facteur R de ressemblance est supérieur à une valeur prédéterminée, par exemple égale à 20

En mettant en oeuvre une comparaison par calcul d'un facteur de ressemblance qui nécessite des ressources de calcul limitées, le procédé conforme à l'invention conduit à un temps de calcul faible. Avantageusement, le spectre mesuré sera collecté et/ou comparé au spectre de référence seulement sur une plage limitée, par exemple entre 550 et 650 nanomètres. Restreindre le domaine d'analyse permet en effet d'exclure l'éventuelle luminescence résiduelle du verre, et, comme expliqué plus haut, de s'affranchir des variations d'épaisseur de la paroi du contenant. En outre, restreindre le domaine d'analyse permet de réduire encore le temps de calcul.

L'ensemble et le procédé conformes à l'invention permettent d'atteindre l'objectif voulu d'un dispositif portable simple et rapide d'utilisation. En normalisant les spectres par rapport aux maxima d'intensité, on s'affranchit à la fois des dispersions liées à la température du contenu et à la forme du contenant, et on réduit les capacités de calcul requises.

De plus, l'architecture et le fonctionnement du dispositif permettent de limiter au maximum le nombre de composants. Ainsi, grâce au choix d'une source lumineuse d'excitation émettant à une longueur d'onde déterminée, on évite l'emploi de filtres afin de sélectionner une bande étroite de longueurs d'onde. En optimisant l'intensité et la qualité du signal de fluorescence, notamment par le choix de la longueur d'onde de la source laser, on peut utiliser une source laser de faible puissance, par exemple de quelques milliwatts. Cela permet de prévoir un dispositif autonome, pouvant fonctionner sur batteries ou piles, et d'éviter de prévoir un dispositif de refroidissement. A cet égard, on notera que le choix d'un capteur de type CCD pour le module de spectrométrie évite également de prévoir un quelconque refroidissement.

## Revendications

1. Ensemble comprenant un contenant (2) au moins partiellement transparent et un dispositif (1) portable de contrôle d'une boisson alcoolisée (26) stockée dans ledit contenant (2), ledit dispositif comportant

   - une source lumineuse (32) unique, émettant un faisceau lumineux d'excitation monochromatique de longueur d'onde comprise entre 350 et 650 nanomètres ;
   - un diviseur de faisceau (36) orienté à 45° par rapport à la direction d'émission de la source lumineuse (32) pour réfléchir le faisceau lumineux d'excitation ;
   - une lentille de focalisation et de collection (40) ;
   - un dispositif de filtrage (44) pour filtrer le rayonnement de fluorescence capté par la lentille de focalisation et de collection (40) et transmis par le diviseur de faisceau (36), de façon à éliminer les longueurs d'ondes inférieures ou égales à la longueur d'onde du faisceau lumineux émis par la source lumineuse ;
   - un module de spectrométrie (50) pour produire un signal correspondant au spectre mesuré du rayonnement de fluorescence de la boisson ;
   - un boîtier (10) contenant ladite source lumineuse (32), ledit diviseur de faisceau (36), ledit dispositif de filtrage (44) et ledit module de spectrométrie (50),

- un dispositif de positionnement (12), solidaire dudit boîtier (10) permettant d'orienter le faisceau lumineux issu de la source lumineuse (32) selon une direction sensiblement normale à la surface externe du contenant (2), le dispositif de positionnement (12) comportant une cale de positionnement formant saillie depuis l'une des faces dudit boîtier (10), ladite cale de positionnement présentant une surface de contact (14) de forme complémentaire du contenant (2) et définissant un espacement entre la lentille de focalisation et de collection (40) et le contenant (2) déterminé de sorte que le faisceau réfléchi (38) est focalisé à l'intérieur du contenant, au sein de la boisson devant être authentifiée contenue dans le contenant (2) à une distance (B) d'une surface interne du contenant inférieure à 1 millimètre et de préférence inférieure à 500 micromètres, le dispositif de positionnement (12) comportant une ouverture traversante pour le passage du faisceau lumineux issu de la source lumineuse ;
- un module d'analyse (62) pour comparer le spectre mesuré à un spectre de référence.

2. Ensemble selon la revendication 1, dans lequel le diviseur de faisceau (36) est un filtre dichroïque, notamment un filtre dichroïque passe-haut relativement aux longueurs d'onde.

3. Ensemble selon la revendication 1 ou 2, dans lequel le dispositif de filtrage comporte un filtre coupe-bande (44) de type Notch et/ou un filtre passe-haut relativement aux longueurs d'onde.

4. Ensemble selon l'une des revendications précédentes, dans lequel le module de spectrométrie (62) est relié au dispositif de filtrage (44) par l'intermédiaire d'une fibre optique (48).

5. Ensemble selon l'une des revendications précédentes, comportant un dispositif d'affichage (70), permettant notamment d'afficher le résultat de la comparaison entre le spectre mesuré et le spectre de référence.

6. Ensemble selon l'une quelconque des revendications précédentes, dans lequel le dispositif de positionnement (12) est connecté au boitier (10) par l'intermédiaire d'une liaison flexible, la lentille de focalisation (40) étant intégrée au dispositif de positionnement (12), la liaison flexible comportant une fibre optique pour transmettre les faisceaux lumineux traversant la lentille de focalisation (40).

7. Ensemble selon l'une des revendications 5 à 6, dans lequel le module d'analyse (62) et le dispositif d'affichage (70) sont inclus dans un dispositif portable annexe (72), tel qu'une tablette informatique ou un téléphone mobile.

8. Ensemble selon l'une des revendications précédentes, comportant des moyens de connexion à une base de données distante.

9. Système de contrôle d'une boisson alcoolisée, le système comportant un ensemble selon la revendication 8 et une base de données stockée dans un serveur distant du dispositif.

10. Système selon la revendication 9, dans lequel la base de données comporte un ou plusieurs spectres de référence pouvant être téléchargés par le dispositif.

11. Procédé de contrôle d'une boisson alcoolisée à travers un contenant au moins partiellement transparent d'un ensemble selon l'une quelconque des revendications 1 à 8, le procédé comportant les étapes consistant à :

- acquérir (80) un spectre de fluorescence de la boisson à travers la paroi du contenant ;
- normaliser le spectre mesuré par rapport au maximum d'intensité d'un spectre de référence ;
- normaliser le spectre de référence par rapport à l'intensité maximum du spectre de référence en prenant pour base 100 l'intensité maximale du spectre de référence,
- calculer un facteur de ressemblance entre le spectre mesuré normalisé et le spectre de référence normalisé ;
- déterminer (86), en fonction de la valeur du facteur de ressemblance obtenue si la boisson est authentique,

ledit procédé étant mis en oeuvre à l'aide du dispositif de l'ensemble selon l'une quelconque des revendications 1 à 8 ou du système selon l'une des revendications 9 et 10.

12. Procédé selon la revendication 11, dans lequel le facteur de ressemblance est calculé sur une plage de longueurs d'onde prédéterminée, par exemple une plage de longueurs d'onde comprises entre 550 et 650 nanomètres.

13. Procédé selon l'une des revendications 11 et 12, dans lequel le facteur de ressemblance est calculé par la méthode dite des moindres carrés ou par un algorithme du type de l'algorithme de Hausdorff.

14. Procédé selon l'une des revendications 11 à 13, dans lequel le spectre de référence est obtenu à partir de la mesure du spectre de fluorescence mesuré sur un échantillon d'une pluralité de contenants au moins partiellement transparents renfermant la boisson authentique.

15. Procédé selon l'une des revendications 11 à 14, comportant une étape de téléchargement du spectre de référence depuis une base de données distante.

**Patentansprüche**

1. Anordnung, die einen mindestens teilweise transparenten Behälter (2) umfasst und eine tragbare Vorrichtung (1) zur Kontrolle eines alkoholischen Getränks (26), das im Behälter (2) gelagert ist, wobei die Vorrichtung beinhaltet:

   - eine einzelne Lichtquelle (32), die einen monochromatischen Anregungslichtstrahl mit einer Wellenlänge zwischen 350 und 650 Nanometern emittiert;
   - einen Strahlteiler (36), der in einem Winkel von 45° zur Emissionsrichtung der Lichtquelle (32) ausgerichtet ist, um den Anregungslichtstrahl zu reflektieren;
   - eine Sammellinse (40);
   - eine Filtervorrichtung (44) zum Filtern der Fluoreszenzstrahlung, die von der Fokussier- und Sammellinse (40) eingefangen wurde, und durch den Strahlteiler (36) übertragen wird, um Wellenlängen zu entfernen, die kleiner oder gleich der Wellenlänge des von der Lichtquelle emittierten Lichtstrahls sind;
   - ein Spektroskopiemodul (50) zum Erzeugen eines Signals, das dem gemessenen Spektrum der Fluoreszenzstrahlung des Getränks entspricht;
   - ein Gehäuse (10), das die Lichtquelle (32), den Strahlteiler (36), die Filtervorrichtung (44) und das Spektroskopiemodul (50) enthält;
   - eine Positionierungsvorrichtung (12), die fest mit dem Gehäuse (10) verbunden ist, und ermöglicht, den von der Lichtquelle (32) ausgehenden Lichtstrahl in einer Richtung auszurichten, die im Wesentlichen senkrecht zur äußeren Oberfläche des Behälters (2) ist, wobei die Positionierungsvorrichtung (12) einen Positionierungskeil beinhaltet, der aus einer der Seiten des Gehäuses (10) herausragt, wobei der Positionierungskeil eine zum Behälter (2) komplementär geformte Kontaktoberfläche (14) aufweist und einen Abstand zwischen der Fokussier- und Sammellinse (40) und dem Behälter (2) definiert, der so bestimmt ist, dass der reflektierte Strahl (38) im Innern des Behälters fokussiert wird, innerhalb des zu authentifizierenden Getränks, das im Behälter (2) enthalten ist, in einer Distanz (B) von einer inneren Oberfläche des Behälters von weniger als 1 Millimeter und vorzugsweise weniger als 500 Mikrometern, wobei die Positionierungsvorrichtung (12) eine durchgehende Öffnung für die Durchkehrung des Lichtstrahls beinhaltet, der von der Lichtquelle ausgeht;
   - ein Analysemodul (62) zum Vergleichen des gemessenen Spektrums mit einem Referenzspektrum.

2. Anordnung nach Anspruch 1, wobei der Strahlteiler (36) ein dichroitisches Filter ist, insbesondere ein wellenlängenbezogenes dichroitisches Hochpassfilter.

3. Anordnung nach Anspruch 1 oder 2, wobei die Filtervorrichtung ein Bandsperrfilter (44) vom Typ Notch beinhaltet und/oder ein wellenlängenbezogenes Hochpassfilter.

4. Anordnung nach einem der vorhergehenden Ansprüche, wobei das Spektroskopiemodul (62) über eine Glasfaser (48) an die Filtervorrichtung (44) angeschlossen ist.

5. Anordnung nach einem der vorhergehenden Ansprüche, eine Anzeigevorrichtung (70) beinhaltet, die insbesondere die Anzeige des Ergebnisses des Vergleichs zwischen dem gemessenen Spektrum und dem Referenzspektrum ermöglicht.

6. Anordnung nach einem der vorhergehenden Ansprüche, wobei die Positionierungsvorrichtung (12) über eine flexible Verbindung mit dem Gehäuse (10) verbunden ist, wobei die Fokussierlinse (40) in die Positionierungsvorrichtung (12) integriert ist, wobei die flexible Verbindung eine Glasfaser beinhaltet, um die Lichtstrahlen zu übertragen, die die Fokussierlinse (40) durchqueren.

7. Anordnung nach einem der Ansprüche 5 bis 6, wobei das Analysemodul (62) und die Anzeigevorrichtung (70) in einer eingegliederten tragbaren Vorrichtung (72) enthalten sind, wie einem Tablet oder einem Mobiltelefon.

8. Anordnung nach einem der vorhergehenden Ansprüche, Mittel zum Verbinden mit einer entfernten Datenbank beinhaltend.

9. System zur Kontrolle eines alkoholischen Getränks, wobei das System eine Anordnung nach Anspruch 8 beinhaltet und eine Datenbank, die in einem von der Vorrichtung entfernten Server gespeichert ist.

10. System nach Anspruch 9, wobei die Datenbank ein oder mehrere Referenzspektren beinhaltet, die von der Vorrichtung heruntergeladen werden können.

11. Verfahren zur Kontrolle eines alkoholischen Getränks durch einen mindestens teilweise transparenten Behälter einer Anordnung nach einem der Ansprüche 1 bis 8, wobei das Verfahren die Schritte beinhaltet, die bestehen aus:

   - Erfassung (80) eines Fluoreszenzspektrums des Getränks durch die Behälterwand hindurch;
   - Normalisierung des gemessenen Spektrums in Bezug auf die maximale Intensität eines Referenzspektrums;
   - Normalisierung des Referenzspektrums in Bezug auf die maximale Intensität des Referenzspektrums, indem als Basis 100 die maximale Intensität des Referenzspektrums genommen wird;
   - Berechnen eines Ähnlichkeitsfaktors zwischen dem normalisierten gemessenen Spektrum und dem normalisierten Referenzspektrum;
   - Bestimmen (86), basierend auf dem erhaltenen Wert des Ähnlichkeitsfaktors, ob das Getränk authentisch ist;

   wobei das Verfahren unter Verwendung der Anordnungsvorrichtung nach einem der Ansprüche 1 bis 8 oder des Systems nach einem der Ansprüche 9 und 10 durchgeführt wird.

12. Verfahren nach Anspruch 11, wobei der Ähnlichkeitsfaktor über einen vorbestimmten Wellenlängenbereich berechnet wird, beispielsweise einen Wellenlängenbereich zwischen 550 und 650 Nanometern.

13. Verfahren nach einem der Ansprüche 11 und 12, wobei der Ähnlichkeitsfaktor durch die Methode, der kleinsten Quadrate genannt, berechnet wird oder durch einen Algorithmus vom Typ Hausdorff-Algorithmus.

14. Verfahren nach einem der Ansprüche 11 bis 13, wobei das Referenzspektrum aus der Messung des Fluoreszenzspektrums erhalten wird, das an einer Probe aus einer Vielzahl von mindestens teilweise transparenten Behältern gemessen wird, die das authentische Getränk einschließen.

15. Verfahren nach einem der Ansprüche 11 bis 14, einen Schritt zum Herunterladen des Referenzspektrums aus einer entfernten Datenbank beinhaltend.

**Claims**

1. An assembly comprising a container (2) at least partially transparent and a portable device (1) for controlling an alcoholic beverage (26) stored in said container (2), said device including

   - a single light source (32) emitting a monochromatic excitation light beam having a wavelength comprised between 350 and 650 nanometers;
   - a beam splitter (36) oriented at 45° with respect to the direction of emission of the light source (32) to reflect the excitation light beam;
   - a focus and collection lens (40);
   - a filtering device (44) for filtering the fluorescence radiation captured by the focus and collection lens (40) and transmitted by the beam splitter (36), so as to eliminate the wavelengths which are shorter than or equal to the wavelength of the light beam emitted by the light source;
   - a spectrometer module (50) for producing a signal corresponding to the measured spectrum of the fluorescence radiation of the beverage;
   - a casing (10) containing said light source (32), said beam splitter (36), said filtering device (44) and said spectrometer module (50),
   - a positioning device (12), secured to said casing (10) making it possible to orient the light beam coming from the light source (32) in a direction substantially normal to the outer surface of the container (2), the positioning device (12) including a positioning wedge forming a projection from one of the faces of said casing (10), said positioning wedge having a contact surface (14) of complementary shape to the container (2) and defining a spacing between the focus and collection lens (40) and the container (2) determined so that the reflected beam (38) is focused inside the container, within the beverage to be authenticated contained in the container (2) at a distance (B) of an internal surface of the container of less than 1 millimeter and preferably less than 500 micrometers, the positioning device (12) including a through opening for the passage of the light beam coming from the light source;
- an analysis module (62) for comparing the measured spectrum with a reference spectrum.

2. The assembly according to claim 1, wherein the beam splitter (36) is a dichroic filter, in particular a high-pass dichroic filter with respect to the wavelengths.

3. The assembly according to claim 1 or 2, wherein the filtering device includes a Notch-type band-stop filter (44) and/or a high-pass filter with respect to the wavelengths.

4. The assembly according to any of the preceding claims, wherein the spectrometer module (62) is connected to the filtering device (44) via an optical fiber (48).

5. The assembly according to any of the preceding claims, including a display device (70), making it possible in particular to display the result of the comparison between the measured spectrum and the reference spectrum.

6. The assembly according to any one of the preceding claims, wherein the positioning device (12) is connected to the casing (10) via a flexible connection, the focus lens (40) being integrated to the positioning device (12), the flexible connection including an optical fiber for transmitting the light beams passing through the focus lens (40).

7. The assembly according to any of claims 5 to 6, wherein the analysis module (62) and the display device (70) are included in an appended portable device (72), such as a tablet computer or a mobile telephone.

8. The assembly according to any of the preceding claims, including connection means to a remote database.

9. A system for controlling an alcoholic beverage, the system including an assembly according to claim 8 and a database stored in a remote server of the device.

10. The system according to claim 9, wherein the database includes one or more reference spectra which can be downloaded by the device.

11. A method for controlling an alcoholic beverage through an at least partially transparent container of an assembly according to any one of claims 1 to 8, the method including the steps of:

- acquiring (80) a fluorescence spectrum of the beverage through the wall of the container;
- normalizing the measured spectrum with respect to the maximum intensity of a reference spectrum;
- normalizing the reference spectrum with respect to the maximum intensity of the reference spectrum by taking as base 100 the maximum intensity of the reference spectrum,
- calculating a resemblance factor between the normalized measured spectrum and the normalized reference spectrum;
- determining (86), depending on the value of the obtained resemblance factor, whether the beverage is genuine,

said method being implemented using the device of the assembly according to any one of claims 1 to 8 or the system according to any of claims 9 and 10.

12. The method according to claim 11, wherein the resemblance factor is calculated over a predetermined wavelength range, for example a wavelength range comprised between 550 and 650 nanometers.

13. The method according to any of claims 11 and 12, wherein the resemblance factor is calculated by the method called least squares method or by an algorithm of the Hausdorff algorithm type.

14. The method according to any of claims 11 to 13, wherein the reference spectrum is obtained from the measurement of the fluorescence spectrum measured on a sample of a plurality of at least partially transparent containers containing the genuine beverage.

15. The method according to any of claims 11 to 14, including a step of downloading the reference spectrum from a remote database.

**Fig. 1**

**Fig. 2**

**Fig. 3**

**Fig. 4a**

**Fig. 4b**

**Fig. 5**

**Fig. 6**

**Fig. 7**

**Fig. 8**

**EP 3 329 257 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2005001451 A2
- US 20150009490 A1
- US 20070023521 A1
- WO 2014184506 A2
- WO 0233404 A2
- US 20020105642 A1